# EUROPEAN PATENT APPLICATION

(11) **EP 1 250 892 A2**
(43) Date of publication of application: **23.10.2002**
(21) Application number: 02252750.1
(22) Date of filing: 18.04.2002
(51) Int. Cl.: A61B 17/80

(54) **Polyaxial locking plate**

(30) Priority: 20.04.2001 US 285462; 18.03.2002 US 100387
(71) Applicant: DEPUY ORTHOPAEDICS, INC., Warsaw, Indiana 46581 (US)
(72) Inventor: Wack, Michael, Warsaw, IN 46580 (US); Bone, Lawrence B, Buffalo, NY 14215 (US); Sanders, Roy W, Tampa, FL 33606 (US)
(74) Representative: Belcher, Simon James

(57) **Abstract**

A fracture repair system for engagement with a bone includes a plate (14), which has a body portion (16) and an internal wall (20) defining a plate hole (22) through the body portion. The system includes one or more bushings (24) having a radially exterior surface (26) and an opposite radially interior surface (30) defining a passageway. The exterior surface of the bushings and the interior wall of the plate are configured to permit polyaxial rotation of the bushings within the plate hole. The system further includes an attachment component (34) having a distal portion (36) sized for clearance passage through the passageway (32) and into the bone and an opposite proximal portion (40) sized to press the bushings against the internal wall of the plate to form a friction lock between the bushings and the plate in a selected polyaxial position. The attachment component is positionable in an orientation extending divergently from the centre of the plate.

## Description

The present invention relates to a bone locking plate, more particularly the present invention relates to a bone locking plate that includes an adjustable attachment component. Most particularly, the present invention relates to a bone locking plate that includes an attachment component whose angle relative to the locking plate may be manipulated during surgery so that an accompanying screw extends into the bone in a desirable orientation.

The skeletal system includes many long bones which extend from the human torso. These long bones include the femur, fibula, tibia, humerus, radius and ulna. These long bones are particularly exposed to trauma from accidents and as such often are fractured during such trauma and may be subject to complex devastating fractures.

Automobile accidents for instance are a common cause of trauma to long bones. In particular the femur and tibia frequently fracture when the area around the knee is subjected to a frontal automobile accident.

Often the distal and/or proximal portions of the long bone, for example, the femur and tibia are fractured into several components and must be re-attached.

Mechanical devices most commonly in the form of pins, plates and screws are commonly used to attach fractured long bones. The plates, pins and screws are typically made of a durable material compatible with the human anatomy, for example titanium, stainless steel or cobalt chrome. The plates are typically positioned longitudinally along the periphery of the long bone and have holes or openings through which screws may be inserted into the long bone transversely. Additionally, intramedullary nails or screws may be utilized to secure fractured components of a long bone, for example, to secure the head of a femur.

Fractures of long bones typically occur in high stress areas, for example, near the condyles or distal or proximal portions of the long bones. Such fractures in the distal or proximal condyle portions of the long bone may result in many individual fragments which must be reconnected. Optimally, the bone plates should be positioned adjacent to the distal or proximal portions of the long bones and permit the securing of these fragments.

More recently bone plates have been provided for long bones which have a profile which conforms to the distal or proximal portion of the long bone. For example such bone plates are available from DePuy ACE in the form of supra condylar plate systems. These plates have a contoured periphery to match the distal portion of a long bone, for example, a femur. These plates, however, include holes or opening through which transverse screws are used to secure the bone plate to the long bone. The openings in the bone plate provide thus for only one general orientation of the screw for attachment of the bone fragments, which is normally or perpendicularly to the bone plate. Thus often the optimum position of a screw may not be utilized as it does not conform to a position nominal or perpendicular to the bone plate.

Often with a fracture of condyles of the distal portion of a long bone the adjacent screws should be positioned and locked in a divergent direction diverging from the bone plate so that the distal condyles may be properly secured by the bone screw. Two dimensional bone plates do not provide for the optimum diverging orientation of the bone screws.

US-5954722 discloses locking plates for use in spinal applications which include a pivotable bushing within the plate which bushing is internally threaded and mates with external threads on bone screws. This type of locking plate permits an orientation of the bone screw in a position other than normally with the bone plate while also permitting locking of the screw.

What is needed is a bone plate assembly that permits the stress free securing of condylar distal or proximal portions of a long bone to a locking plate and allows for diverging locking orientation of adjacent bone screws within a bone plate to optimally secure the fragments from a long bone trauma.

In one aspect, the invention provides a fracture repair system for engagement with a bone. The system includes a plate. The plate has a body portion and an internal wall defining a plate hole through the body portion. The system also includes a bushing having a radially exterior surface and an opposite radially interior surface defining a passageway. The exterior surface of the bushing and the interior wall of the plate are configured to permit polyaxial rotation of the bushing within the plate hole. The system also includes an attachment component having a distal portion sized for clearance passage through the passageway and into the bone and an opposite proximate portion sized to press the bushing against the internal wall of the plate to form a friction lock between the bushing and the plate in a selected polyaxial position. The attachment component is positionable in an orientation extending divergently from the centre of the plate.

In another aspect, the present invention provides a knee fracture repair system for engagement with a bone which includes a femur plate including a body portion conforming at least partially to the contour of the femur and an internal wall defining a femur plate hole through the body portion. The system also includes a tibia plate including a body portion conforming at least partially to the contour of the tibia and an internal wall defining a tibia plate hole through the body portion. The system further includes a bushing including a radially exterior surface and an opposite radially interior surface defining a passageway. The exterior surface of the bushing and the interior wall of the plate are configured to permit polyaxial rotation of the bushing within at least one of the femur plate holes and the tibia plate holes. The system also includes an attachment component including a distal portion sized for clearance passage through the passageway and into the bone and an opposite proximate portion. The proximate portion is sized to press the bushing against the internal wall of the plate to form a friction lock between the bushing and at least one of the femur plate holes and the tibia plate holes in a selected polyaxial position.

In a further aspect, the invention provides a femur plate for use in a knee fracture repair system for engagement with a bone. The femur plate includes a plate portion having a body portion conforming at least partially to the contour of the femur and an internal wall defining a femur plate hole through the body portion. The femur plate also includes a bushing including a radially exterior surface and an opposite radially interior surface defining a passageway. The exterior surface of the bushing and the interior wall of the plate portion are configured to permit polyaxial rotation of the bushing within the plate hole. The femur plate includes an attachment component including a distal portion sized for clearance passage through the passageway and into the bone and an opposite proximate portion. The proximate portion is sized to press the bushing against the internal wall of the plate portion to form a friction lock between the bushing and the plate portion in a selected polyaxial position. The attachment component is positionable in an orientation extending divergently from the centre of the plate portion.

In yet another aspect, the invention provides a tibia plate for use in a knee fracture repair system for engagement with a bone. The tibia plate includes a plate portion having a body portion conforming at least partially to the contour of the tibia and an internal wall defining a tibia plate hole through the body portion. The tibia plate also includes a bushing having a radially exterior surface and an opposite radially interior surface defining a passageway. The exterior surface of the bushing and the interior wall of the plate are configured to permit polyaxial rotation of the bushing within the plate hole. The tibia plate further includes an attachment component having a distal portion sized for clearance passage through the passageway and into the bone and an opposite proximate portion. The proximate portion is sized to press the bushing against the internal wall of the plate to form a friction lock between the bushing and the plate in a selected polyaxial position. The attachment component is positionable in an orientation extending divergently from the centre of the plate.

In another aspect, the invention provides a method for coupling two bone portions together. The method includes the step of providing a locking plate apparatus including a plate having a body portion and at least two plate holes through the body portion, a bushing movably coupled in each plate hole and having a radially exterior surface, an opposite interior surface, and first and second ends defining a passageway between them, and at an attachment component for each bushing sized for extension into the passageway. Each attachment component includes opposite leading and trailing portions. The method also includes the steps of positioning the body portion upon the bone portions so that the plate holes in the plate are situated over bone and rotating at least one bushing within the plate hole until the first and second ends of the bushing are aligned along an axis that extends through a pre-determined portion of the bone. The method also includes the steps of inserting the leading portion of the attachment components into the respective passageway and driving the trailing portion of each attachment component through the respective passageway until the leading portion is positioned in the bone and the exterior surface of the bushing is pressed against the body portion to form a friction lock between them.

The invention will now be described by way of example with reference to the accompanying drawings, in which:
Fig. 1 is a plan lateral view of a fracture repair system in accordance with the present invention with a femur plate coupled to a femur and a tibia plate coupled to a tibia;
Fig. 2 is a perspective lateral view of a fracture repair system in accordance with the present invention with a femur plate coupled to a femur and a tibia plate coupled to a tibia;
Fig. 3 is a perspective view of a femur plate in accordance with the present invention;
Fig. 4 is a plan view of a the femur plate of Figure 3;
Fig. 5 is an enlarged plan view of a the femur plate of Figure 3;
Fig. 6 is a cross section view of the femur plate of Figure 5 taken along lines 6-6;
Fig. 6A is a cross section view of the femur plate of Figure 5 taken along lines 6A-6A;
Fig. 7 is a perspective view of a tibia plate in accordance with the present invention;
Fig. 8 is a plan view of the tibia plate of Figure 7;
Fig. 9 is a cross-section view of the tibia plate of Figure 8 taken along lines 9-9;
Fig. 10 is a plan view of a cannulated, cancellous bone screw for attachment to cancellous bone of a long bone for use with the fracture repair system of Figure 1;
Fig. 11 is a partial cross sectional view of the tibia plate of Figure 8 taken along lines 11-11 showing a portion of the tibia plate coupled to the tibia with the tibia plate in cross section and showing various bone screws positioned in the femur;
Fig. 12 is a partial cross sectional view of the femur plate of Figure 4 taken along lines 12-12 showing a portion of the femur plate coupled to the femur with the femur plate in cross section and showing two of the bone screws of Figure 14 positioned in divergent positions in the condyles of the femur in accordance with the present invention;
Fig. 12 A is a partial cross sectional view of the femur plate of Figure 4 taken along lines 12A-12A showing a portion of the femur plate coupled to the femur with the femur plate in cross section and showing the bone screw of Figure 10 positioned in the condyles of the femur;
Fig. 13 is a plan view of a cortical bone screw for attachment to both cortical bone surfaces of a long bone and for engagement with the bone plate for use with the femur plate of Fig. 4;
Fig. 14 is a plan view of a bone screw for engagement with the bone plate installed in a bone plate according to the present invention with a portion of the bone plate and a bushing providing polyaxial rotation shown in cross section;
Fig. 15 is a top view of a bushing for providing polyaxial rotation of the bone screw according to the present invention;
Fig. 16 is a plan view shown in cross section of the bushing of Fig. 15;
Fig. 17 is a plan view of a drill guide instrument installed on a bone plate for use with the fracture repair system of Figures 1-16; and
Fig. 18 is a plan view of a cancellous bone screw for attachment to cancellous bone of a long bone for use with the fracture repair system of Figure 1.

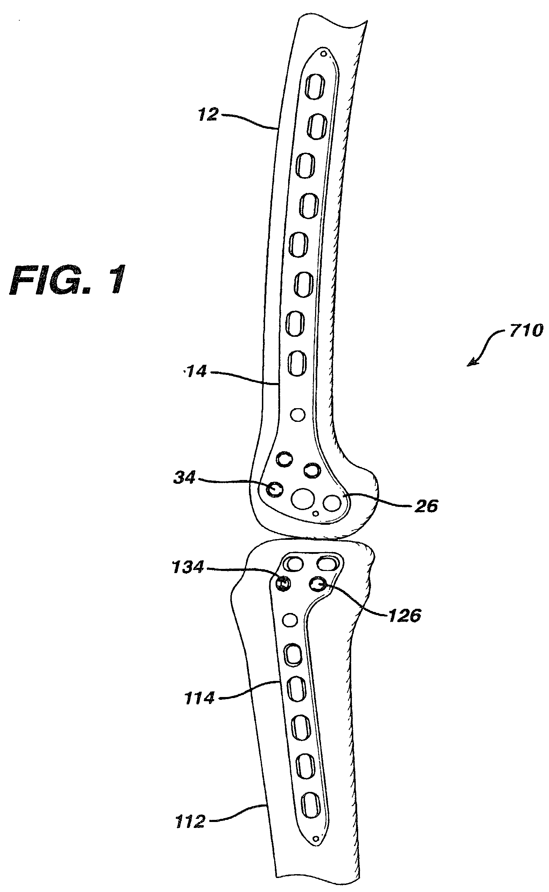

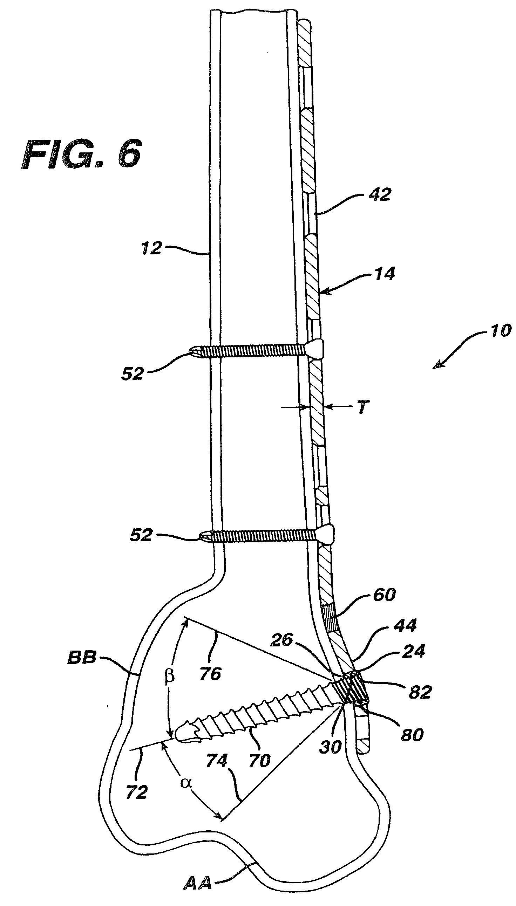

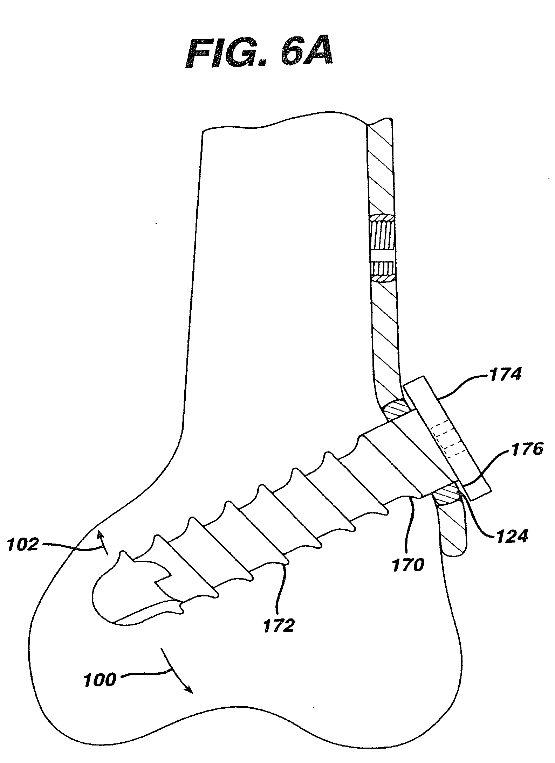

Referring to the drawings, Figure 6 shows a fracture repair system 10 for engagement with a long bone 12. The long bone 12 may be any long bone, for example, a femur, tibia, fibula, humerus, radius or ulna, but as shown in Figure 1 the long bone is a femur. The fracture repair system 10 includes a plate 14.

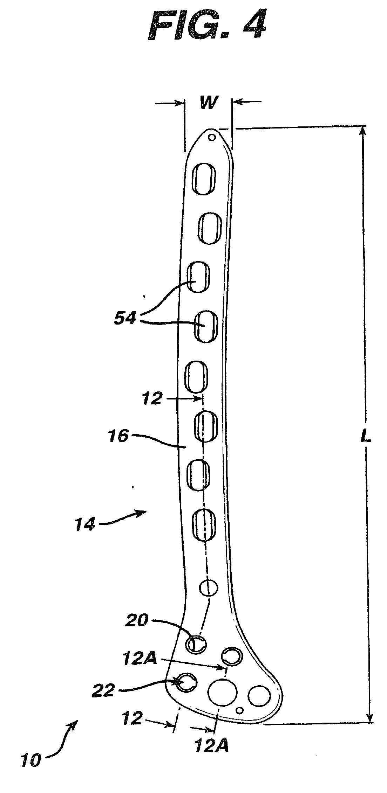

Referring now to Figure 4 the fracture repair system 14 is shown in greater detail. The plate 14 may be made of any suitable durable material and may, for example, be made of a metal, for example, a metal compatible with the human anatomy, for example, cobalt chrome, stainless steel or titanium. The plate 14 includes a body portion 16 and an interior wall 20. The interior wall 20 defines a plate hole 22 through the body portion 16.

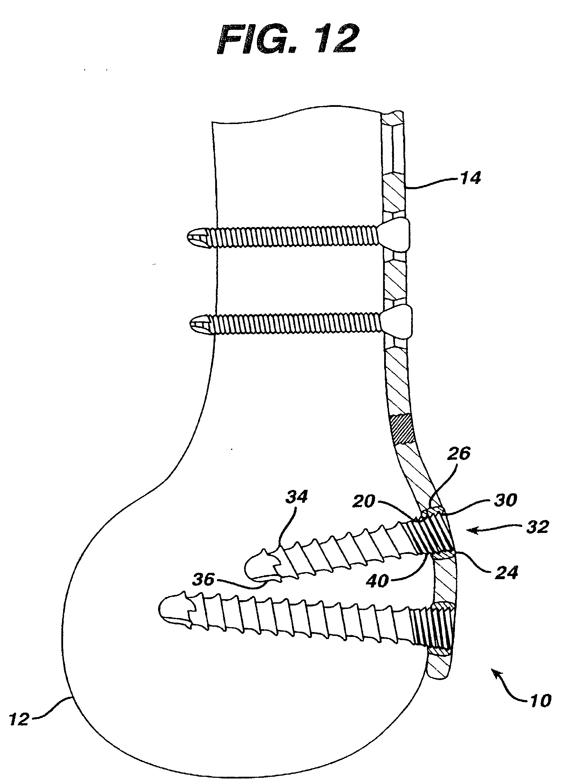

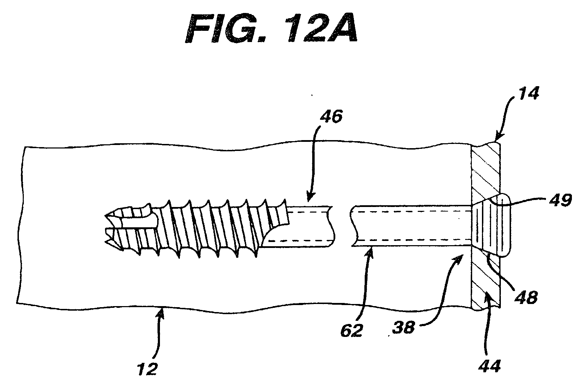

Referring now to Figure 12 the fracture repair system 10 is shown in greater detail. In addition to the plate 14 the fracture repair system 10 includes one or more bushings 24. The bushing 24 includes a radial exterior surface 26 and opposite radial interior surface 30. The opposite radial interior surface 30 defines a passageway 32 through the bushing 24. The exterior surface 26 of the bushing 24 and the interior wall 20 of the plate 14 are configured to permit the polyaxial rotation of the bushing 24 within the plate hole 22 (see Figure 12). Such polyaxial rotation may be permitted by providing an arcuate or spherical surface on the interior wall 20 of the plate 14 and a mating arcuate or spherical surface on the radial exterior surface 26 of the bushing 24.

The fracture repair system 10 further includes the attachment component 34 includes a distal portion 36 sized for current passage through the passageway 32 and into the long bone 12. The attachment component 34 further includes an opposite proximal portion 40 sized to press the bushing 24 against the internal wall 20 of the plate 14 to form a friction lock between the bushing 24 and the plate 14 in a selected polyaxial position. The attachment component 34 is positionable in an orientation extending divergently from the centre of the plate.

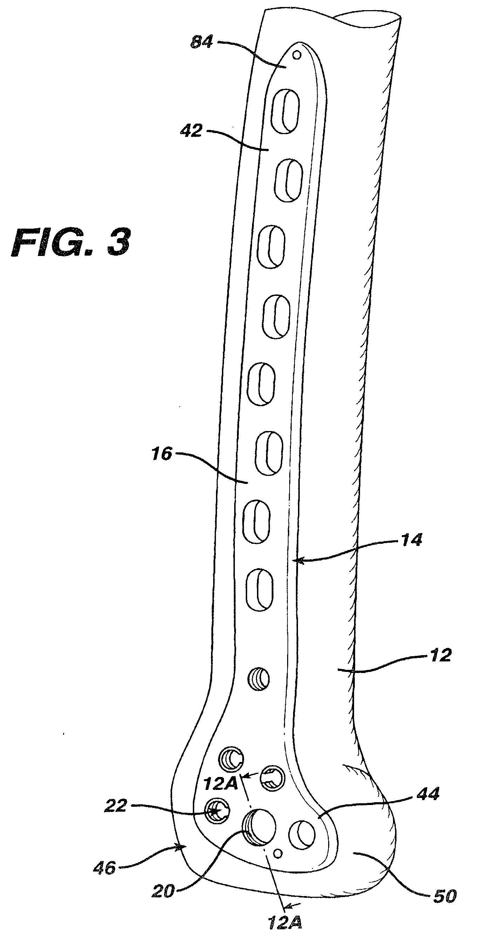

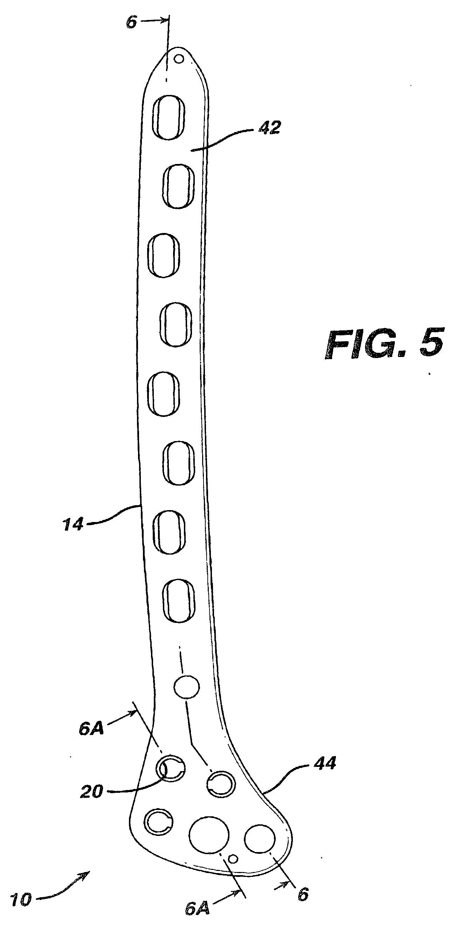

Referring now to Figures 3 through 6 the fracture repair system 10 is shown as femur plate assembly 10. Preferably and as shown in Figures 3-6, the body portion 16 of the femur plate 14 preferably includes a proximal portion 42 and distal portion 44. To provide for optimal support of the femur, the femur plate 14 has a shape generally conforming to the outer periphery of the femur 12. The proximal portion 42 of the bone plate 14 is generally flat or planer in conforming to the general flat or planer nature of the proximal shaft portion of the femur. The femur plate 14 may have a bow to accommodate the natural anterior/posterior bow of the femur 12. The distal portion 44 of the femur plate 14 has a shape generally conforming to the condylar portion 46 of the femur 12. Since the condylar portion 46 of the femur 12 is arcuate or curved the distal portion 44 of the femur plate 14 is preferably curved to mate with condyles 50 of the condyle portion 46 of the femur 12.

While the particular size and shape and dimensions of the femur plate 14 may vary widely depending upon the size of the femur on which it is installed, for an adult human femur, the plate 14 may have, for example as shown in Figure 4, an overall length L of about 15.2 to 35.5 cm (6 to 14 inch) and a width W of about 0.64 to 1.9 cm (0.25 to 0.75 inch) or 0.9 to 3.17 cm (0.75 to 1.25 inch) and a thickness T of, for example as shown in Figure 6, about 0.31 to 0.64 cm (0.125 to 0.25 inch). Since the human anatomy is generally symmetrical, the femur plate 14 is either a right hand or left hand femur plate and the right hand and left-hand femur plates are different, but generally symmetrical with each other.

While the fracture repair system of the present invention includes one or more bushings which cooperates with an attachment component such that the attachment component may be positionable in an orientation diverging from the centre of the plate, it should be appreciated that the fracture system or plate may include a plurality of attachment components. Further these attachment components may be of different styles or types.

Referring now to Figure 6, the femur plate 14 is shown with three different types of attachment components. Solid, fully-threaded, cortical screws 52 are positioned in elongated openings 54 shown in Figure 4 in the proximal portion 42 of the femur plate 14. The fully-threaded cortical screws 52 may, as shown in Figure 6, be self-tapping and cut threads while they are being screwed through the plate into the bone during surgery. The cortical screws 52 are supported primarily by the cortical bone to which they have been secured. While the proximal portion 42 of the bone plate 14 may be secured by a solitary cortical screw 52 preferably and, as shown in Figure 6, the proximal portion 42 of the femur plate 14 is supported by a series of several spaced apart fully threaded cortical bone screws.

To provide ample support for the proximal portion 42 of the plate 14 and to provide for a standard commercially available femur plate 14, the femur plate 14 preferably includes a uniformly spaced apart pattern of elongated openings 54 shown in Figure 4. The surgeon may choose any of a number of the elongated openings 54 shown in Figure 4 in which to drill and screw the cortical screws. Depending on the position of the fractures as few as two or three cortical screws may be sufficient to support the femur plate 14.

Continuing to refer to Figure 6, cancellous screws 56 may also be placed in the elongated openings 54 and used to secure the proximal portion 42 of the femur plate 14.

The screw 56 unlike cancellous screw 70 (see Figure 6) does not include threads on the head of screw 56. The lack of screw threads on the head of screw 56 allows the head to spin on the bushing 24 without locking, thereby achieving a lagging effect. The cancellous screws 56 may be any suitable size and may, for example, be 2 to 6 mm solid, cancellous, partially or fully threaded cancellous screws. The cancellous screws 56, preferably have a length less than the thickness of the femur so that they may not protrude from the opposite surface of the femur.

Distal portion 44 of the femur plate 14 is designed to follow the general contours of the lateral distal femur while the proximal portion 42 incorporates the natural bow of the femur.

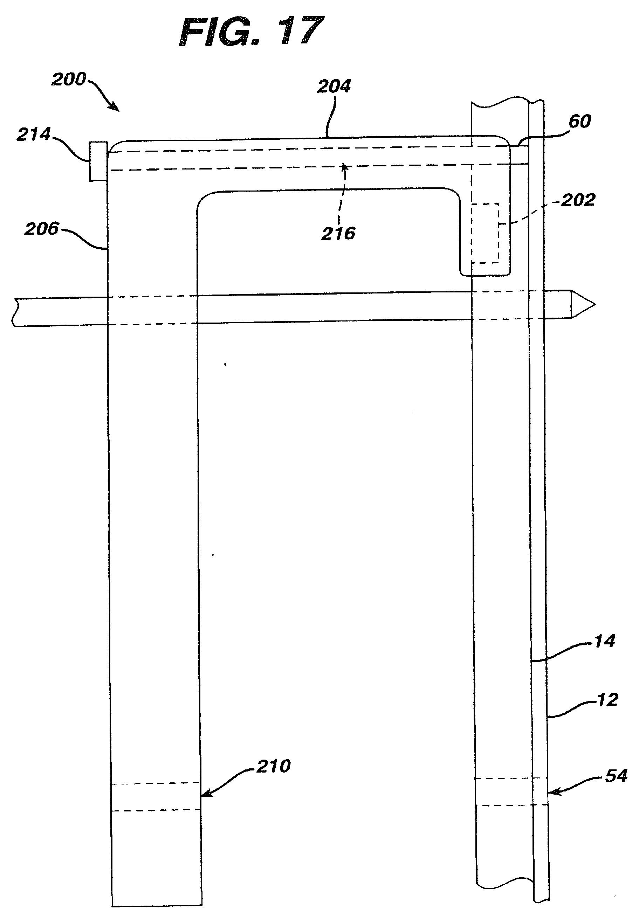

The femur plate 14 may include one or more tapped openings 60 in the femur plate 14 which may be utilized to secure a drill guide 200 shown in Figure 17 for aligning a drill and a screw driver for the insertion of the screws 52 and 56 into the femur. The drill guide 200 will be described in greater detail later.

According to the present invention, the plate 14 includes attachment components which are positionable in an orientation diverging from the centre of the plate. The plate 14 thus includes at least one screw 70 which is secured to the plate 14 by means of the bushing 24. The screw 70 may be in the form of a cancellous screw. The cancellous screw is particularly well suited for securing the condylar portion of the distal portion of the femur. The cancellous screw 70 may be partially or fully threaded and may have any suitable length to reach the proper portion of the fractured condylar portion of the distal femur. For example, the cancellous screw may have a length from 20 to 150 millimetres. The cancellous screw may have a suitable diameter to properly secure the fractured portions of the femur. For example, the cancellous screw may have a diameter of 3 to 10 millimetres. The cancellous screw 70 is used to secure the distal portion of the femur plate to the bone.

The cancellous screws may be rotated from the first position 72 shown in solid to position 74 shown rotated an angle α or to a third position 76 rotated in the opposite direction an angle β (see Figure 6). If the cancellous screw 70 is rotated to the second position 74, the screw 70 will be utilized to secure fragment AA while, if the cancellous screw 70 is rotated to position 76, the cancellous screw 70 may be utilized to secure fragment BB. The tip of the cancellous screws 70 can therefore be rotated in a conical pattern.

The cancellous screw 70 as shown in Figure 6 may include external threads 80 on the head or proximal portion of the screw 70. Alternatively, the head or proximal portion may have a smooth conical head. The external threads 80 mate with internal threads 82 on the bushing 24. Preferably and as shown in Figure 6 the external threads 80 are tapered such that as the external threads 80 of the screw 70 are engaged into the bushing 24 the bushing 24 expands, locking the radial external surface 26 of the bushing 24 to the radial interior surface 30 of the plate 14.

By permitting the bushing 24 to rotate within the plate 14 and by permitting the bushing 24, the screw 70 and the plate 14 to all be locked securely in place, the screw may be fixedly positioned in many different orientations, while maintaining all components at minimal stress. As shown in Figure 6, the feature of having the positionable screw and plate configuration permits either fragment AA or fragment BB to be secured by the screw 70.

Referring now to Figure 6A, one or any portion of the locations of the plate 14 may include one or more bushings 124 which may alternatively be utilized with a screw having a non-threaded head. For example, as shown in Figure 6A a cancellous screw 170 is shown similar to screw 70 but not including external threads on the head of the screw. The screw 170 does include cancellous threads 172 for securement to the cancellous bone. The screw 170 includes a head 174 which is secured against face 176 of bushing 124. In this configuration the bushing 124 serves to permit the rotation of the screw 170 in the direction of arrows 100 and 102, thus permitting the orientation of the screw 170. The use of the bushing 124 prevents stress risers on the head 174 or face 176 of the bushing 124.

The plate 14 may be made of any suitable durable material that is biologically compatible with the human anatomy and preferable made of a high strength metal. For example, the plate may be made of stainless steel, cobalt chrome or titanium. Preferably the plate 14 is manufactured from a forged or wrought titanium alloy. One such suitable alloy complies with test method ASTM F-620-97 and another suitable alloy complies with test method ASTM F-136 ELI.

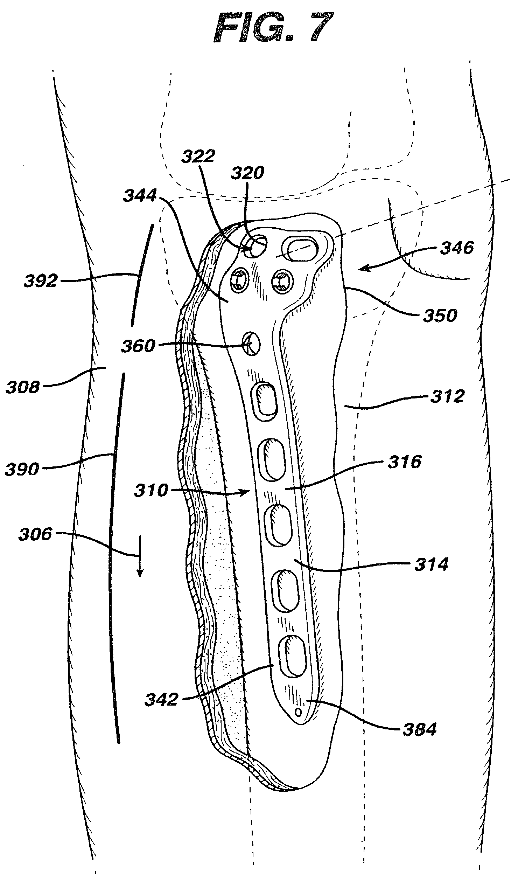

Referring to Figure 7, the femoral plate 314 may be secured to the femur 312 during surgery either percutaneously or by conventional open surgery. When the femur plate 314 and screws are implanted in conventional open surgery a longitudinal cut 390 is made through the skin along the thigh 308 laterally where the femur plate is typically installed. A lateral installation of the femur plate provides for the minimal interference with muscle, ligaments and other soft tissue. A longitudinal cut 306 in the thigh 308 through the skin parallel to the femur 312 is made approximately the length of the femur plate 314 and the soft tissue is pulled apart so that the femur plate may be placed in position. Cancellous and cortical screws are then positioned over their respective openings in the femur plate 314 and secured to the femur 312.

When performing percutaneous surgery the skin of the thigh 308 is opened laterally near the knee and a transverse cut 392 is made and femur plate 314 is inserted at that opening and guided against the femur 312 proximally toward the hip. The proximal end of the femur plate 314 may include a contoured tip 384 to ease the percutaneous installation of the femur plate 314.

While the femur plate 314 may be made of any suitable size depending on the size of the human in which the plate is to be installed, the femoral plates 314 may be available in various lengths so that they will be available when trauma strikes. For example, the femoral plates may be provided with varying lengths including for example 5, 8, 11, 14 or 18 screw holes in the shaft.

The cortical and cancellous screws are manufactured of any suitable durable material that are typically manufactured of a wrought titanium alloy for example which complies with test method ASTM F-136 ELI.

Referring now to Figure 12A, the femur plate 14 may further include a cannulated cancellous screw for positioning in the condylar portion 46 of the femur 12. The cannulated cancellous screw 62 preferably has a length slightly shorter than the length of the portion of the cancellous bone at the condylar portion 46 such that the cannulated cancellous screw does not contact the opposite cortical bone. The cannulated cancellous screw may, for example, be 8 mm cannulated cancellous and preferably as shown in Figure 12A include external threads 48 located on proximal portion 38 of the cancellous screw 46. The external threads 48 mate with the internal threaded opening 49 of the distal portion 44 of the femur plate 14. The cannulated cancellous screw 62 provides additional structural support to the condylar portion 46 of the femur 12. Alternatively, the head of the cancellous screw 62 may be smooth, thereby allowing the head to spin in the plate without locking. The spinning achieves a lagging effect, i.e. drawing the fragments together.

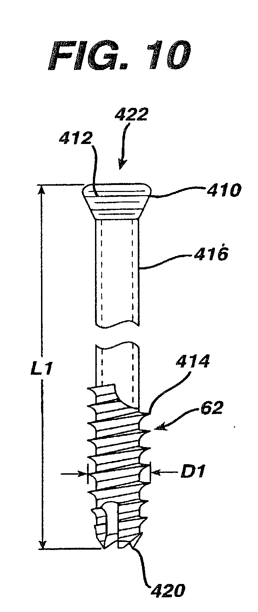

Referring now to Figure 10, the cannulated cancellous screw 62 is shown in greater detail. The cannulated cancellous screw may not be in any particular size and may include a diameter D1 of, for example, 4 to 10 millimetres. The cannulated cancellous screw has a length L1 sufficient to occupy most of the condylar portion 46 of the femur or long bone 12. To provide for rigid attachment of the cannulated cancellous screw 62 to the bone plate 14, the cancellous screw 62 preferably includes a head 410 having external threads 412 which may mate with internal threaded opening 49 of the bone plate 14 (see Figure 12A). The cannulated cancellous screw 62 includes external threads 414 and may include an unthreaded shank portion 416. The cannulated cancellous screw 62 may include a self-tapping tip 420 which may also serve as a self-drilling as well as a self-tapping tip. As shown in Figure 10, the threads 412 on the head 410 are tapered to provide for a tight locking fit with the bone plate 14. The cannulated cancellous screw 62 is by definition cannulated or includes a central longitudinal opening 422.

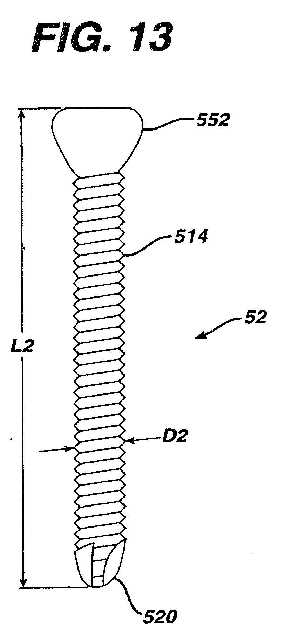

Referring now to Figure 13 a cortical screw 52 is shown. The cortical screw 52 includes threads 514 which are adapted for securing cortical bone. The cortical screw 52 may include an unthreaded shank portion (not shown). The cortical screw 52 includes a head 552 which may, as shown in Figure 13, have a generally oval shape. The cortical screw 52 may also include a self-tapping tip 520 which may also include self-drilling provisions. The cortical screw 52 has a length L2 which preferably is of sufficient length to engage the cortical bone on the opposite or exit side of the bone. The cortical screw 52 further includes a thread diameter D2 which is of sufficient size to provide sufficient holding power and engagement with the cortical bone. For example and as shown in Figure 13, the cortical screw 52 has a diameter D2 of, for example, 3.5 to 6 millimetres.

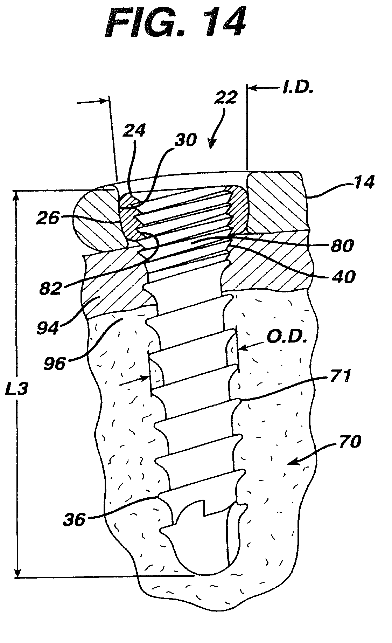

Referring now to Figure 14 an attachment component according to the present invention is showed as cancellous screw 70. The screw 70 includes a distal portion 36 which has an outside diameter OD which is less than the inside diameter ID of the internal wall or surface 30 of the plate hole 22. The screw 70 further includes external threads 80 located on the proximal portion 40 of the screw 70. Preferably and as shown in Figure 14, the external threads 80 are tapered. The external threads 80 are mateably engageable with the internal threads 82 on the bushing 24. The bushing 24 is pivotally engageable with the plate 14. The radially exterior surface 26 of the bushing 24 has a generally spherical shape and is mateably fitted with the interior wall or surface 30 of the plate hole 22. The interior threads 82 of the bushing 24 is larger than the outside diameter of cancellous threads 71 on the screw 70 to permit the distal portion of the 36 of the screw 70 to slidably pass or thread through the plate hole 22. The cancellous threads 71 are adapted for efficient engagement with cancellous bone 96 and the screw 70 has a length L3 which is sized to provide for the cancellous thread 71 to engage a significant portion of the cancellous bone 96.

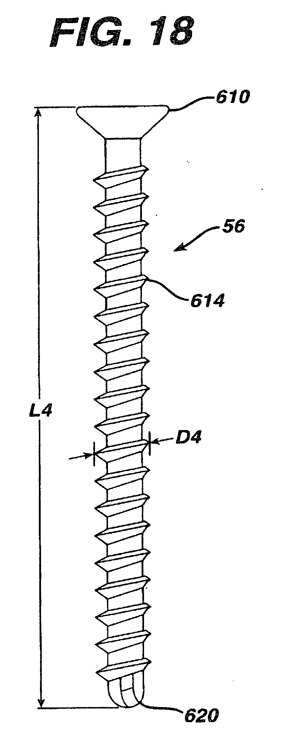

Referring now to Figure 18, a fully threaded cancellous screw 56 is shown for use with the bone plate 14. The cancellous screw 56 includes a head 610. The head 610 may have any suitable shape and may, for example, be flat head as shown in Figure 18 or have a pan head shape. The screw 56 unlike cancellous screw 70 (see Figure 6) does not include threads on the head of screw 56. The lack of screw threads on the head of screw 56 allows the head to spin on the bushing 24 without locking, thereby achieving a lagging effect. The cancellous screw 56 has a length L4 to provide for engagement with a suitable portion of the cancellous bone (not shown). The cancellous screw has threads 614 which are adapted for engagement with cancellous bone. The thread 614 has a diameter D4 which is sized for efficient and effective support and engagement with the cancellous bone. For example, the cancellous screw 56 may have a diameter D4 of, for example 2 to 6 mm. The cancellous screw 56 further includes a tip 620. The tip 620 may optionally include self-drilling and/or self-tapping features.

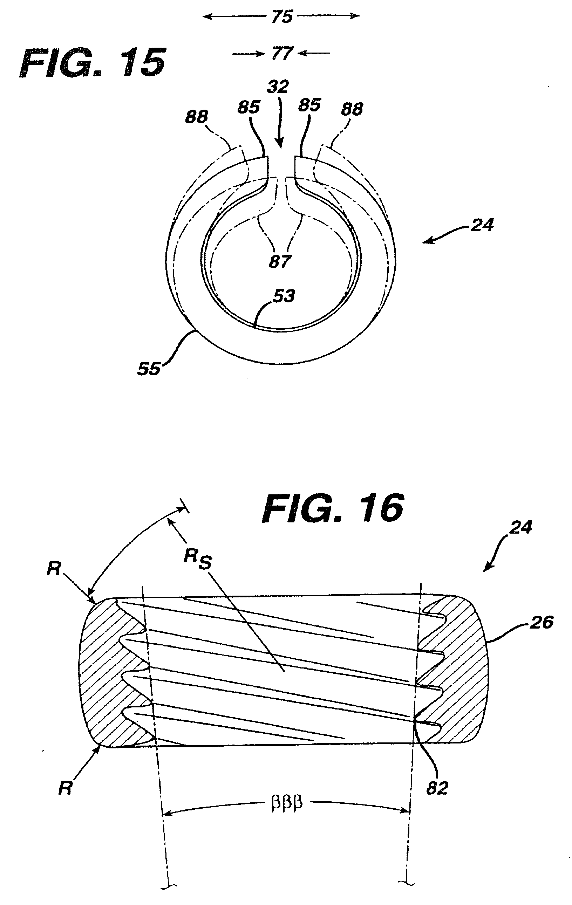

Referring now to Figure 15, the bushing 24 is shown in greater detail. The bushing or collet is manufactured of any suitable durable material that is compatible with the human body. For example the collet may be made of cobalt chrome, stainless steel or titanium. For example the bushing 24 may be manufactured of a wrought titanium alloy. A suitable wrought titanium alloy is such as complies with test method ASTM F-136 ELI.

The bushing 24 preferably includes a radial opening or passageway 32 on the periphery of the bushing 24. The passageway 32 extends from the radially exterior surface 55 through the opposite radially interior surface 53. The bushing 24 has a first relaxed position 85 which represents the shape of the bushing 24 when not assembled into the plate 14. The bushing 24 further has an assembled position 87 as shown in the dotted line. The assembled position 87 represents when the bushing 24 is placed within the plate 14 and when the screws are not installed. The bushing 24 further has an expanded position 88 shown in phantom in which the bushing 24 is shown with the bushing 24 installed in the plate 24 and the screws installed within the bushing 24.

As can be seen in Figure 15, the bushing 24 is contracted when the assembled position 87 to provide for an interference fit between the bushing 24 and the plate 14. Further as shown in Figure 15, the bushing 24 is expanded as it moves from the assembled position 87 to the expanded position 88. This occurs because the tapered threads during engagement cause the bushing 24 to enlarge. The enlarging of the bushing 24 causes a tighter interference between the bushing 24 and the plate thereby securely locking the bushing in its polyaxial oriented position with minimal stress.

Referring now to Figure 16 a cross-section of the bushing 24 is shown. As shown in Figure 16 preferably the bushing 24 has a spherical radius Rₛ which defines the radial exterior surface 26 of the bushing 24. By providing a spherical radius Rₛ the bushing 24 may be oriented into a number of angular positions with respect to the plate.

Referring to Figure 16 the internal threads 82 of the bushing 24 have a taper defined by an internal angle βββ. The angle βββ may be, for example, from 3 to 30°. As shown in Figure 16 the truncated spherical shape of the radial exterior surface 26 may be modified by corner radius R.

While the fracture repair system of the present invention includes the bushing to provide for positioning of the attachment component in a variety of diverging directions while providing for reduced stress at the plate, when percutaneously securing a bone screw to a bone plate location which does not provide for the pivotal securement of the bushing arrangement, it is critical that the screws in such fixed locations be properly positioned. Referring now to Figure 17 preferably the femur plate 14 is used in conjunction with drill guide 200. Drill guide 200 is installed onto the femur plate 14 during surgery and is utilized to guide drills and screwdrivers to properly orientate the screws that are placed in the proximal portion of the plate 14. The drill guide 200 includes a locating feature 202 in the form of, for example, an elongated pin which closely fits to the elongated slots of a plate. The drill guide includes a riser portion 204 and a bar portion 206 which is positioned parallel and spaced from the plate 14.

The bar portion 206 includes a series of bushing holes 210 which are in alignment with the centre of the elongated openings 54 in the plate 14. To properly secure the drill guide 200 to plate 14, for example, the drill guide 200 may include a securing screw 214 which may be slidingly fitted to an opening 216 in the riser portion 204 and which may be secured to tapped opening 60 in the plate 14.

The drill guide 200 may be utilized both in conventional open surgery and in percutaneous surgery. When utilized in percutaneous surgery the bushing holes 210 may be utilized to guide trocars which will open the skin and tissue around the openings permitting the screws to be properly secured. Since the human anatomy is generally symmetrical, the drill guide 200 is either a right hand or left hand drill guide and the right hand and left-hand drill guide are different, but generally symmetrical with each other. It should be appreciated that the drill guide may be utilized for any bone plate for supporting any long bone for example a tibia, humerus, ulna, radius or fibula.

While the fracture repair system has been described in detail as a femur plate, it should be appreciated that the plate may be utilized for supporting any long bone for example a tibia, humerus, ulna, radius or fibula.

Referring now to Figures 7 to 9 and 11, a tibia plate 314 for installation onto a tibia 312 is shown. The fracture repair system 310 for use on the tibia 312 includes a tibia plate 314 having a body portion 316. The body portion 316 includes a distal portion 342 and a proximal portion 344. The tibia plate 314 like the femur plate 14 is preferably positioned laterally on the long bone. The lateral position of the tibia plate reduces the amount of soft tissue that must be dislocated to position the tibia plate 314. Since the human anatomy is generally symmetrical, the tibia plate 314 is either a right hand or left hand tibia plate and the right hand and left-hand tibia plates are different, but generally symmetrical with each other. The proximal portion 344 of the tibia plate 314 is designed to follow the general contours of the lateral proximal tibia. The proximal portion 344 of the plate 314 is contoured to fit the lateral condyle 350 of the condylar portion 346 of the tibia 312. The body portion 316 of the tibia plate 314 like the body portion 16 of the femur plate 14 has a generally arcuate cross-section to conform with the distal shaft of the tibia 312.

The tibia plate 314 like the femur plate 14 may be made of any suitable durable material that is compatible with the human immune system and may for example be made of a durable non-corrosive material such as stainless steel, cobalt chrome or titanium. For example, the tibia plate may be manufactured from a forged or wrought titanium alloy. For example, such a titanium alloy may be such as complies with test methods ASTM F-620-97 or ASTM F-136 ELI.

Referring now to Figure 7, the tibia plate 314 may be inserted into the human anatomy percutaneously or by conventional open surgery. When inserted by conventional open surgery, the leg 308 is cut with a longitudinal incision 390 of length roughly equal to that of the tibia plate 314. The soft tissue is moved away from the tibia 312 and the tibia plate 314 is placed against the tibia 312. Screws such as those for the femur plate are utilized to secure the tibia plate 314 to the tibia 312. If the tibia plate 314 is to be inserted percutaneously, a smaller longitudinal incision 392 is made in the skin of the leg 308 near the knee and the distal portion 342 of the body portion 316 of the tibia plate 314 is inserted in the incision 392 in the direction of arrow 306 toward the distal portion of the leg. A contoured tip 384 on the distal portion of the 342 of the tibia plate of the tibia plate 314 is shaped to ease the insertion of the tibia plate along the contour of the tibia 312 in the direction of arrow 306.

For installation either percutaneously or by conventional open surgery of the tibia plate 314 drill guides (not shown) such as drill guide 200 for the femur plate as shown in Figure 17 are utilized. Again, as with the femur plate, the drill guide may be utilized to guide the drill and the screws whether the plate and screws are inserted percutaneously or by conventional open surgery. It should be appreciated that a left-hand drill guide (not shown) and a right hand drill guide (not shown) are necessary respectively for the right hand and left-hand tibia plates (not shown).

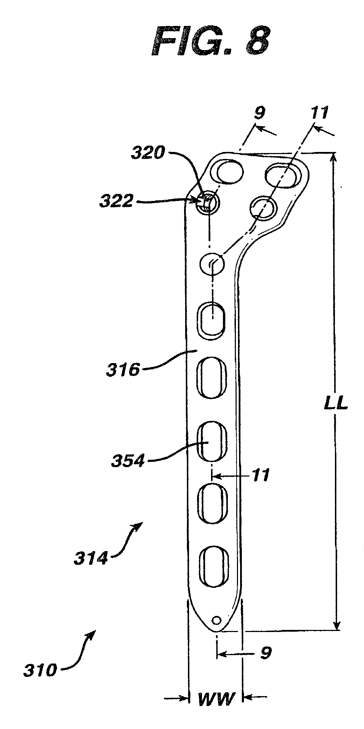

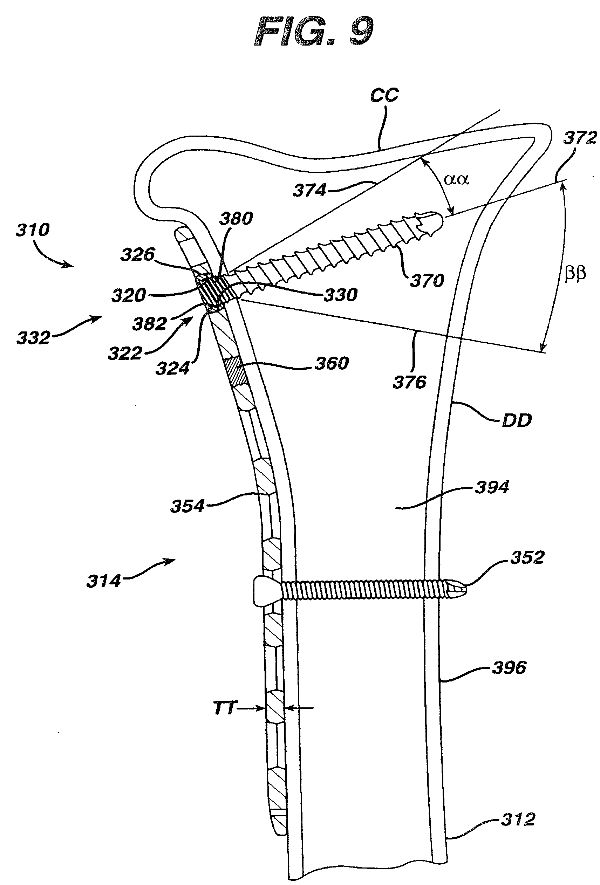

Referring now to Figure 8, the tibial plate 314 may be made of sufficient dimensions to properly support the tibia 312. The proper dimensions of the tibial plate 314 are dependent thus on the size of the particular tibia to be treated as well as the inherent strength of the material from which the tibial plate 314 is made. For example, the tibial plate 314, if made of titanium, may have a thickness TT (see Figure 9) of, for example, approximately 1.6 to 6.4 mm (0.063 to 0.25 inch) and a WW width of around 6.4 mm to 19 mm (0.25 to 0.75 inch) and a length LL of, for example, from 12.7 to 25.4 cm (5 to 10 inch). To provide for a range of standard tibial plates, the tibial plates may be provided in varying lengths of, for example, a length with a number of elongated openings 354 of, for example, 4, 7, 11, or 14 elongated openings.

According to the present invention and referring to Figures 7 to 9 and 11, the tibial plate 314 includes the body portion 316 which conforms at least partially to the contour of the tibia 312. The tibia plate 314 also includes an interior wall 320 which defines a tibia plate hole 322 through the body portion 316.

Referring to Figure 9, the tibial plate 314 further includes one or more bushings 324. The bushing 324 includes a radially exterior surface 326 and an opposite radially interior surface 330. The opposite radially interior surface 330 defines a passageway 332 there through. The exterior surface 326 of the bushing 324 and the interior wall 320 (see Figure 9) of the plate 314 cooperate with each other and are configured to permit polyaxial rotation of the bushing 324 within the plate hole 322. The tibial plate 314 further includes an attachment component 370 in the form of, for example, a cancellous screw. The screw 370 includes a distal portion 336 sized for clearance passage through the passageway 332 and into the cancellous bone 394.

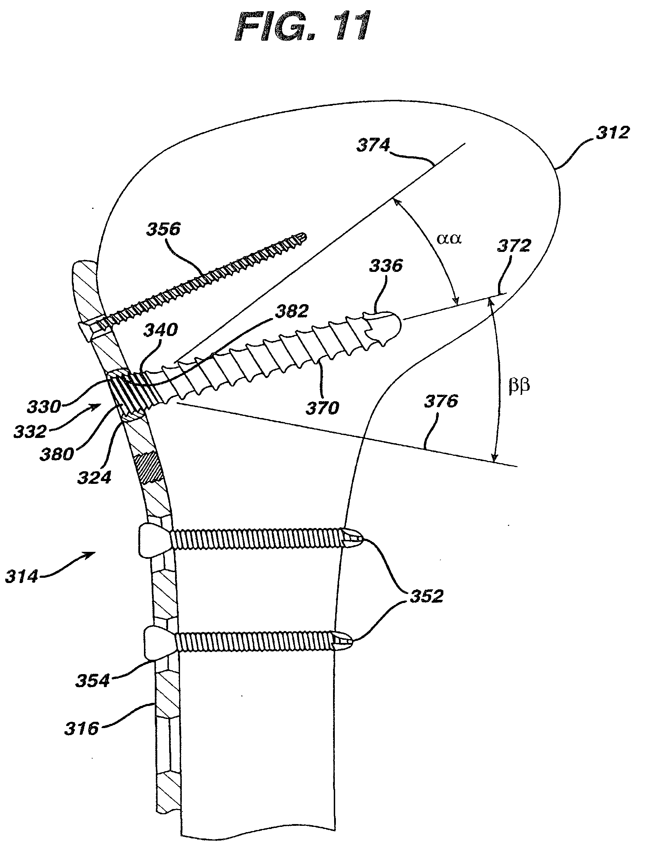

The screw 370 further includes a proximate portion 340 sized to press the bushing 324 against the inner wall or surface 330 of the plate 324 to form a friction lock between the bushing 324 and the plate 314 in a selected polyaxial position. For example, the cancellous screw 370 may be in a first polyaxial position 372 as shown in solid line 372 (see Figure 11). Alternatively, the cancellous screw 370 may be oriented an angle αα from the first position 372 into a second position 374 as shown in phantom. Alternatively, the cancellous screw 370 may be positioned in, for example, a third position 376 positioned at an angle ββ from the first position 372. The cancellous screw 370 may thus be positioned with a diverging angle αα or ββ from the first position 372.

Preferably and as shown in Figure 11, the proximal portion 340 of the cancellous screw 370 includes external tapered threads 380 which mate with internal threads 382 located within the bushing 324. By providing tapered threads as the cancellous screw 370 is screwed into the bushing 324, the bushing 324 expands with the radially exterior surface 326 of the bushing, seating and securing against the radially interior surface 330 of the plate 314. This provides for stress-free, secure locking of the screw 370 to the plate 314.

Alternatively, the attachment component which mates with the bushing 324 may be provided without any threads in the proximal portion of the attachment component similarly to the screw 170 of Figure 6A. Such a screw will provide for polyaxial positioning of the attachment component with reduced stress. The screw 56 unlike cancellous screw 70 (see Figure 6) does not include threads on the head of screw 56. The lack of screw threads on the head of screw 56 allows the head to spin on the bushing 24 without locking, thereby achieving a lagging effect.

By positioning the cancellous screw 370 into the first position 372 or the second position 374 or the third position 376, the screw 370 may be positioned to properly secure fragments. For example as shown in Figure 9 the cancellous screw 370 being positioned in second position 374 may provide for the securing of a fragment CC while the positioning of the cancellous screw 370 in the third position 376 may provide for the securing of fragment DD.

The fracture repair system 310 for use for repairing a fractured tibia may include additional attachment components such as additional attachment component 370. Thus the fracture repair system may include a second cancellous screw 370 positioned at a second plate hole (not shown). In addition to a plurality of cancellous screws 370, the fracture repair system 310 may include, in addition to the polyaxial screws, additional cancellous or cortical screws. For example, Referring to Figures 9 and 11, the repair system 310 may include fully threaded cortical screws 352 similar to the cortical screws 52 of the femur plate 14. The cortical screws 352 preferably extend through the cancellous bone 394 and engage with the cortical bone 396. The fracture repair system 310 may further include cancellous screws for example cancellous screws 356 located in the proximal portion 344 of the bone plate 314 as shown in Figure 11. Such cancellous screws 356 are preferably of a length short enough that they do not reach through to the opposed cortical bone 396. The tibial plate 314 may include one or more tapped openings 360 in the tibial plate 314 which may be utilized to secure a drill guide (not shown), similar to the drill guide 200, for aligning a drill and a screw driver for the insertion of the screws 352 into the tibia.

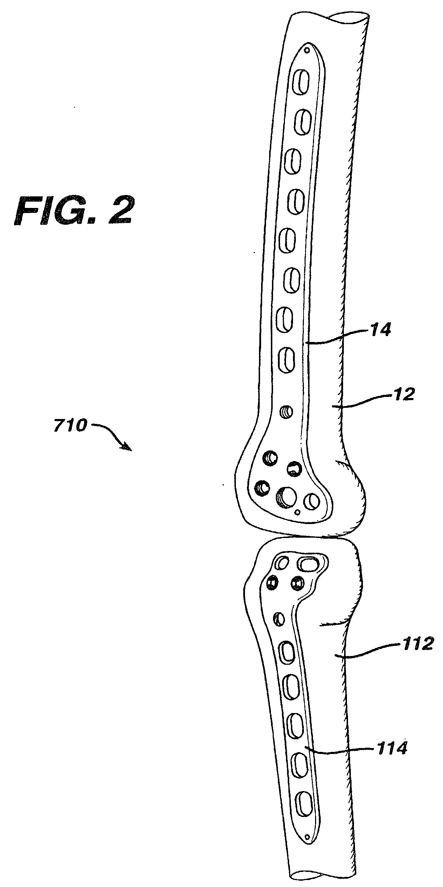

Referring now to Figures 1 and 2 a fracture repair system 710 comprises an assembly of both a femur plate 14 and a tibia plate 114. Frequently the polyaxial plates of the present invention are sold as a fracture repair system 710 including both a tibial plate 114 and a femur plate 14. Such a combination is often required in severe knee trauma caused, for example, in front-end auto accidents. It should be appreciated that a fracture repair system may include a plate for any other long bone for example a humerus, ulna, fibula or radius.

By providing a fracture repair system including a bushing to permit polyaxial rotation of the bushing within the hole plate an attachment component may be secured to a plate with the ability to position divergently to secure the fracture of the bone most efficiently. For example bone fragments may be reached by orienting the attachment component relative to the plate in such a direction to reach various bone fragments.

By providing a fracture repair system including a bushing with a spherical outside diameter in cooperation with a plate having a spherical bore, a low-friction polyaxial rotation of the attachment component relative to the plate is possible.

By providing a fracture repair system including a bushing having a tapered threaded bore in cooperation with a tapered threaded or non-threaded attachment component, the attachment component may be rigidly secured in a variety of orientations.

By providing a fracture repair system including a polyaxial bushing which may be rigidly secured to a plate and including a closely conforming plate which closely conforms to the condyle areas of a long bone the fragments fractured components within the condyle areas may be effectively and efficiently contained.

By providing a fracture repair system including a threaded alignment hole for securing a jig for drilling and threading the plate to the bone perpendicularly, a simple to use effective efficient bone plate system can be provided.

By providing a bone plate including a contoured tip for percutaneous insertion, a bone plate may be provided percutaneously for minimally invasive surgery. Such a contoured tip permits easy and effective insertion and alignment of the plate to the bone.

## Claims

1. A fracture repair system for engagement with a bone, the system comprising:
a plate including a body portion and an internal wall defining a plate hole through the body portion,
a bushing including a radially exterior surface and an opposite radially interior surface defining a passageway, the exterior surface of said bushing and the interior wall of said plate being configured to permit polyaxial rotation of said bushing within the plate hole
an attachment component including a distal portion sized for clearance passage through the passageway and into the bone and an opposite proximate portion sized to press said bushing against the internal wall of said plate to form a friction lock between said bushing and said plate in a selected polyaxial position, said attachment component being positionable in an orientation extending divergingly from the centre of said plate.

2. A fracture repair system as in claim 1 wherein said plate defines a surface thereof, the surface closely conforming to the bone.

3. A fracture repair system as in claim 1 wherein said plate includes a second internal wall defining a second plate hole, said second internal wall defining internal threads; and in which the system includes a second attachment component defining external threads thereon, said second attachment component threadably secured to said plate.

4. A knee fracture repair system for engagement with a bone, the system comprising:
a femur plate including a body portion conforming at least partially to the contour of the femur and an internal wall defining a femur plate hole through the body portion,
a tibia plate including a body portion conforming at least partially to the contour of the tibia and an internal wall defining a tibia plate hole through the body portion,
a bushing including a radially exterior surface and an opposite radially interior surface defining a passageway, the exterior surface of the bushing and the interior wall of said plate being configured to permit polyaxial rotation of the bushing within at least one of the femur plate hole and the tibia plate hole, and
an attachment component including a distal portion sized for clearance passage through the passageway and into the bone and an opposite proximate portion sized to press the bushing against the internal wall of the plate to form a friction lock between the bushing and at least one of the femur plate hole and the tibia plate hole in a selected polyaxial position.

5. A fracture repair system as in claim 1 or claim 4 wherein said bushing defines an axial opening on the periphery thereof from the radially exterior surface through the opposite radially interior surface

6. A fracture repair system as in claim 1 or claim 4 wherein said attachment component is positionable in an orientation extending divergently from the centre of the plate.

7. A fracture repair system as in claim 1 or claim 4 wherein the plate further comprises a second internal wall defining a second plate hole through the body portion, the system including a second bushing including a radially exterior surface and an opposite radially interior surface defining a second passageway, the exterior surface of the second bushing and the second interior wall of said plate being configured to permit polyaxial rotation of the second bushing within the second plate hole, and further including a second attachment component including a distal portion sized for clearance passage through the second passageway and into the bone and an opposite proximate portion sized to press the second bushing against the internal wall of the plate to form a friction lock between the second bushing and the plate in a selected polyaxial position, said second attachment component being positionable in an orientation extending divergently from the centre of the at least one of the femur plate hole and the tibia plate hole.

8. A fracture repair system as in claim 1 or claim 4 wherein the radially exterior surface of said bushing comprises a truncated spherical shape, and wherein the internal wall defining the plate hole comprises a truncated spherical shape.

9. A fracture repair system as in claim 1 or claim 4 wherein said attachment component comprises first external threads on the proximate portion thereof and second external threads on the distal portion thereof, and wherein the radially interior surface of said bushing comprises first internal threads thereon, said first internal threads of said bushing engageable with said first internal threads of said attachment component.

10. A fracture repair system as in claim 1 or claim 4 wherein at least one of said first internal threads of said bushing and said first internal threads of said attachment component are tapered.

11. A knee fracture repair system as in claim 4 wherein at least one of said femur plate and said tibia plate includes a second internal wall defining a second plate hole, said second internal wall defining internal threads; and which includes a second attachment component defining external threads thereon, said second attachment component threadably secured to said at least one of said femur plate and said tibia plate.
